# EUROPEAN PATENT SPECIFICATION

(11) **EP 0 490 818 B1**
(45) Date of publication and mention of the grant of the patent: **20.08.1997**
(21) Application number: 91810942.2
(22) Date of filing: 05.12.1991
(51) Int. Cl.: A61K 31/70, C07H 19/167

(54) **6-CYCLOHEXYL-2'-0-METHYL-ADENOSINE HYDRATE AND USES THEREOF**
6-CYCLOHEXYL-2'-0-METHYL-ADENOSINE HYDRAT UND VERWENDUNGENGEN DAVON
HYDRATE DE 6-CYCLOHEXYL-2'-0-METHYL-ADENOSINE ET SON UTILISATIONS

(30) Priority: 07.12.1990 DE 4039060; 26.07.1991 GB 9116212
(43) Date of publication of application: 17.06.1992
(60) Divisional application: 96120163.9
(73) Proprietor: Novartis AG, 4058 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Gadient, Fulvio, CH-4127 Birsfelden (CH); Milavec, Maria, F-68300 Village-Neuf (FR); Müller, Else, CH-4436 Oberdorf (CH)

(56) References cited:
- EP-A- 0 179 667
- EP-A- 0 269 574
- EP-A- 0 378 518
- WO-A-88/03147
- WO-A-88/03148
- DE-A- 2 230 160
- GB-A- 2 226 027
- US-A- 4 962 194

## Description

The present invention relates to 6-cyclohexyl-2'-O-methyl-adenosine hydrate of formula A

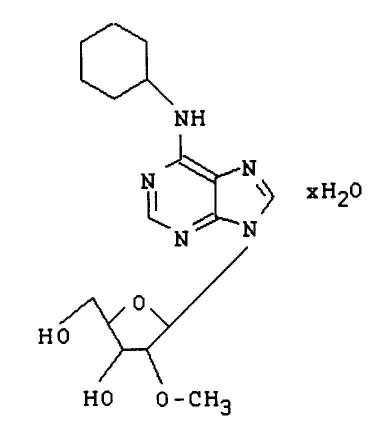

In an other aspect, the invention provides the use of 6-cyclohexyl-2'-O-methyl-adenosine hydrate, for the preparation of medicaments suitable for the treatment of high blood pressure, as coronary vasodilators, for inhibition of both thrombocyte aggregation and activation of leucocytes, for lowering blood lipid levels, against congestive heart failure, myocardial infarction, sudden cardiac death, renal insufficiency further for the treatment of neurodegenerative disorders, certain peripheral neuropathies such as diabetic neuropathy and of disorders associated with peripheral vascular disease and/or disorders associated with neuronal degeneration, hypertriglyceridemia, elevated free fatty acids and/or type I and type II diabetes, arrhythmias and for protection against myocardial infarction.

The present invention also provides a pharmaceutical composition comprising 6-cyclohexyl-2'-O-methyl-adenosine hydrate and a pharmaceutically acceptable carrier therefor.

The hydrate is formed by treatment of the known monoetherate of 6-cyclohexyl-2'-O-methyl-adenosine with an aqueous solvent.

Especially preferred is the 1.5 hydrate form.

The 1.5 hydrate named 6-cyclohexyl-2'-O-methyl-adenosine 1.5 hydrate can be prepared in the following manner: 0,7 g 6-chloro-9-purinyl-2'-O-methyl-D-ribose are heated in 30 ml of cyclohexylamine during 2 hours to 80°. The mixture is subsequently evaporated to dryness under reduced pressure. The residue obtained is chromatographed on silica gel using a mixture of methylenechloride/ethanol 95:5 as eluent. The purified product is crystallized form methylenechloride/diethylether. As the product contains diethylether that can not be removed even in high vacuum it is crystallized from ethanol/water. The thus obtained 6-cyclohexyl-2'-O-methyl-adenosine 1.5 hydrate has a melting point of 88-91°, [α]_{D}²⁰ = -57,2 ° (c = 1 in dimethylformamide).

| **Elementary Analysis** | | |
|---|---|---|
| **Calculated :** | C | 52,3% |
| | H | 7,2% |
| | N | 17,9% |
| | O | 22,5% |
| **Found :** | C | 52,5% |
| | H | 7,2% |
| | N | 18,0% |
| | O | 22,4% |

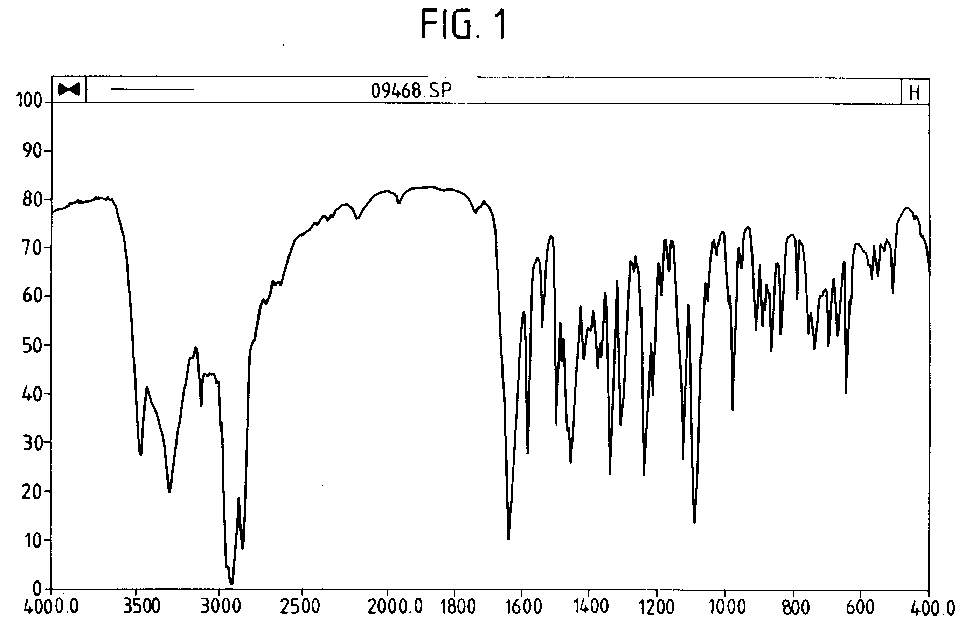

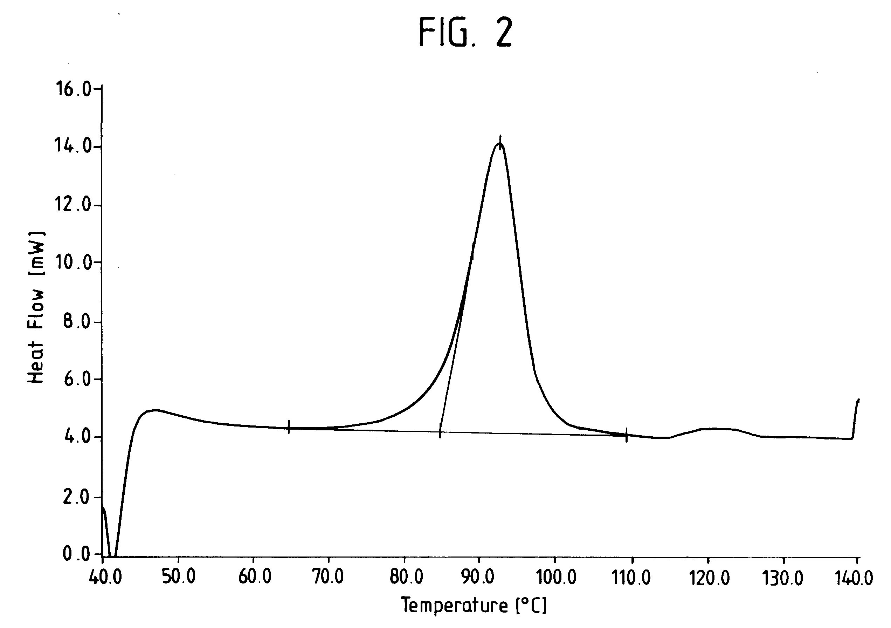

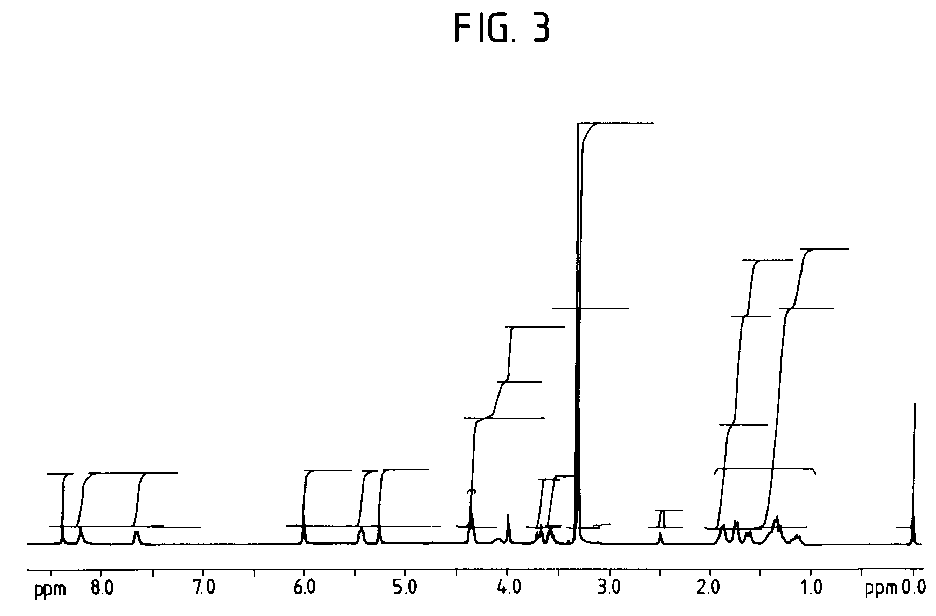

The IR spectrum of this 1.5 hydrate, the result of DSC thermal analysis and the results of NMR analysis are attached as figures 1, 2 and 3. The results of these measurements together with the results of elementary analysis show that according to the above process 6-cyclohexyl-2'-O-methyl-adenosine 1.5 hydrate has been prepared.

The hydrate of this invention may be formulated for administration by any suitable route, the preferred route depending upon the disorder for which treatment is required, and preferably in unit dosage form or in a form that a human patient may administer to himself in a single dosage. Advantageously, the composition is suitable for oral, rectal, topical, parenteral, intravenous or intramuscular administration.

Preparations may be designed to give slow release of the active ingredient.

The compositions of the invention may be in the form of tablets, capsules, sachets, vials, powders, granules, lozenges, suppositories, reconstitutable powders, or liquid preparations such as oral or sterile parenteral solutions or suspensions. Topical formulations are also envisaged where appropriate.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol, or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling, tabletting or the like. Repeated blending operations may be used to distribute the active agent throughout those compositions employing large quantities of fillers.

Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such livid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in polyethyleneglycol or ethanol and diluted with water for injection and filter sterilized before filling into a suitable vial or ampoule and sealing. Advantageously, adjuvants such as a local anaesthetic, a preservative and buffering agents can be dissolved in the vehicle. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10-60% by weight of the active material, depending on the method of administration. 6-cyclohexyl -2'-O-methyl-adenosine hydrate may also be administered as a topical formulation in combination with conventional topical excipients.

Topical formulations may be presented as, for instance, ointments, creams or lotions, impregnated dressings, gels, gel sticks, spray and aerosols, and may contain appropriate conventional additives such as preservatives, solvents to assist drug penetration and emollients in ointments and creams. The formulations may contain compatible conventional carriers, such as cream of ointment bases and ethanol or oleyl alcohol for lotions.

Suitable cream, lotion, gel, stick, ointment, spray or aerosol formulations that may be used for 6-cyclohexyl-2' -O-methyl adenosine hydrate are conventional formulations well known in the art, for example, as described in standard text books of pharmaceutics and cosmetics, such as Harry's Cosmeticology published by Leonard Hill Books, Remington's Pharmaceutical Sciences, and the British and US Pharmacopoeias.

Suitably, 6-cyclohexyl-2' -O-methyl adenosine hydrate will comprise from about 0.5 to 20% by weight of the formulation, favourably from about 1 to 10% for example 2 to 5%.

The dose of the compound used in the treatment of the invention will vary in the usual way with the seriousness of the disorders, the weight of the sufferer, and the relative efficacy of the compound. However, as a general guide suitable unit doses may be 0.1 to 1000 mg, such as 0.5 to 200, 0.5 to 100 or 0.5 to 10 mg, for example 0.5, 1, 2, 3, 4 or 5 mg; and such unit doses may be administered more than once a day, for example 2, 3, 4, 5 or 6 times a day, but preferably 1 or 2 times per day, so that the total daily dosage for a 70 kg adult is in the range of about 0.1 to 1000 mg, that is in the range of about 0.001 to 20 mg/kg/day, such as 0.007 to 3, 0.007 to 1.4, 0.007 to 0.14 or 0.01 to 0.5 mg/kg/day, for example 0.01, 0.02, 0.04, 0.05, 0.06, 0.08, 0.1 or 0.2 mg/kg/day; and such therapy may extend for a number of weeks or months.

When used herein the term "pharmaceutically acceptable" encompasses materials suitable for both human and veterinary use. No toxicological effects have been established for 6-cyclohexyl--2' -O-methyl-adenosine hydrate in the above mentioned dosage ranges. The following pharmacological tests have been effected to establish the activity of the compounds:

### a) Affinity for adenosine receptors

Pig striatal membranes were prepared as previously described by H. Bruns et al. in Molecular Pharmacology 29 (1986) pages 331-344. ³H-NECA, a non-selective adenosine receptor agonist was used to label both A₁ and A₂ receptors. IC₅₀ values were derived from the displacement curves by weighted non-linear least-square curve fitting to the Langmuir equation and pK_{D} values calculated.

The results are presented in the following Table in agreement with the literature, CPA proved a potent and highly selective displacer of binding to the A₁ receptor, CV 1808 was a relatively weak but selective A₂ receptor ligand and CGS 21680 showed high potency and selectivity for the A₂ receptor. 6-cyclohexyl-2' -O-methyl-adenosine hydrate in the form of its 1.5 hydrate shows good affinity and high selectivity for the A₁ receptor.

**Table**

| Affinity of adenosine receptor ligands for A₁ and A₂ receptors | | | | |
|---|---|---|---|---|
| | **A**_{**1**} **(K**_{**D**}**;nM)** | **A**_{**2**} **(K**_{**D**}**;nM)** | **A**_{**1**}**:A**_{**2**} **selectivity** | **n** |
| CPA^{a} | 0.74 ± 0.01 | 33 ± 110 | 1260 | 6 |
| CV 1808^{a} | 2460 ± 757 | 269 ± 70 | 0.1 | 5 |
| CGS 21680^{a} | 4360 ± 1080 | 10 ± 4 | 0.004 | 4 |
| Compound No. 7 1.5 H₂O | 23 ± 2 | 24500 ± 5160 | 1090 | 5 |
| CPA^{a} = Cyclopentyladenosine CV 1808^{a} = 2-Phenylaminoadenosine (Carbohydrates vol. 81 (1974) ref. 91898 K) CGS 21680^{a} = 2-[p-(2-Carboxyethyl)phenethylamino]-5'-N-ethyl carboxamido-adenosine (FASEB J, 1989, 3 (4) Ref. 4770 and 4773) | | | | |

### b) Arrhythmias

Adenosine receptor activation reduces the incidence of supraventricular tachycardia and other arrhythmias. The test method is disclosed by C. Clarke et al. in The Pharmaceutical Journal Vol. 244, 595 to 597 (1990) . In this test 6-cyclohexyl--2' -O-methyl-adenosine hydrate is used in form of its 1.5 hydrate.

### c) Mean arterial blood pressure, bradycardia and peripheral vasodilation on anesthetized rats.

The experiments were carried out on male Wistar rats, body weight 300-350 g (323 ± 3 g), under Inactin anaesthesia (150 mg/kg i.p.), according to the method of Schroeder et al. (Measurement of the cardiodynamics, haemodynamics and the ECG in the anesthetized rat, effects of catecholamines (dopamine and isoproterenol) in Budden et al. "The Rat Electrocardiogram in Pharmacology and Toxicology" Oxford Pergamon Press 1981, 155-9) Catheters were placed in the right jugular and right femoral veins, in the left ventricle (inserted via the right carotid artery), left femoral artery, and aorta (inserted through the right femoral artery). The following variables were measured or calculated: systolic, diastolic, and mean arterial blood pressure (mm Hg; left femoral artery, Statham pressure transducer P 23 Gb), pulse pressure (mm Hg), heart rate (beats/min; triggered from the blood pressure curve), rate of rise of left ventricular pressure (dP/dtₘₐₓ, mm Hg/s; Statham pressure transducer P 23 Gb), cardiac output (ml/min/100 g body weight, thermodilution method, right jugular vein and aorta), total peripheral resistance (dynes · s cm⁻⁵/100 g body weight), ECG lead III. Arterial blood pressure, left ventricular pressure, dP/dt ₘₐₓ, heart rate, and electrocardiogram were continuously recorded with a Schwarzer polygraph. The parameters were measured 30, 20, 10 and 2 min before administration of the substance and 1, 10, 30 and 60 min after injection of the drug into the right femoral vein. 6-cyclohexyl-2' -O-methyl-adenosine hydrate in form of its 1.5 hydrate was tested in doses of 0.003, 0.01 and 0.03 mg/kg whereby five animals per dose were used.

### d) Blood flow through ischaemic skeletal muscle

Male normotensive rats (ca. 300 g) were anaesthetised with Evipan Na (160 mg/kg i.p.) and the right femoral artery was ligated. Three weeks after ligation the rats were anaesthetised with pentothal (Thiopental-Na, 40 mg/kg i.p.) and placed on a special holder for measurement of the resonances of phosphocreatine (PCr), inorganic phosphate (Pi), of the three phosphates of ATP, and of phosphomono-esters and sugar phosphates (PM) by use of a Bruker Biospec 47/15 spectrometer equipped with a a 4.7 T horizontal magnet of 15 cm clear bore. The blood supply to the right hindleg was then temporarily occluded for about 30-40 min by a tourniquet placed around the upper hindleg, until the PCr levels had fallen to about 1/3 of the original level. Thereafter the occlusion was released and the rate of recovery (τ₁) of PCr was followed. Thirty minutes later the same procedure was repeated without and after intravenous administration of the drug e.g. compound No. 7 in form of its 1.5 hydrate under investigation, the rate of recovery or the PCr level (τ₂) being followed again. The ratio of both recovery times (τ₂/τ₁) was taken as a parameter for the drug effect on the peripheral circulation. Some animals were tested with the sympathetic neurotoxin 6-hydroxydopamine, at the time of femoral artery ligation and the effects of 6-cyclohexyl-2' -O-methyl-adenosine hydrate in the form of its 1.5 hydrate on the induced peripheral vascular insufficiency determined.

### e) Glucose Transport in Rat Adipocytes

Adipocytes were isolated from the epididymal fat pads of normal chow-fed rats by digestion with collagenase. Cells (final concentration 2% v/v) were pre-incubated with adenosine deaminase (1 U/ml), 6-cyclohexyl-2' -O-methyl-adenosine hydrate in form of its 1.5 hydrate and other additions as indicated for 30 min at 37°C. [3-³H]Glucose (final concentration, 50 µM, 0.5 µCi/ml) was then added, and incubation was continued for a further 60 min. Incorporation of radioactivity from [3-³H]-glucose into cell lipids (a measure of glucose transport) was evaluated by extraction of the cell suspension (0.5 ml) with 5 ml of toluene-based scintillant, followed by liquid scintillation counting; water-soluble metabolites and residual [3-³H] glucose remain in the aqueous phase and are not detected.

### f) Dyslipidemias characterized by elevated serum triglycerides

Several studies have shown a positive correlation between serum triglyceride levels and the risk for coronary heart disease (CHD) (Grundy, in Cholesterol and Atherosclerosis: Diagnosis and Treatment, Lippincott, Philadelphia (1990)). The value of reducing elevated triglyceride levels as an approach to reducing the risk of CHD emerged from the Helsinki Heart Study where, following treatment with gemfibrozil, the greatest reduction in serious coronary events occured in Type IIB hyperlipidemic patients in whom both LDL-cholesterol and total serum triglycerides were elevated. 6-cyclohexyl-2' -O-methyl-adenosine hydrate in the form of its 1.5 hydrate produced substantial and long-lasting falls in plasma free fatty acids and triglycerides in the Rhesus monkey.

### g) Protection against infarction by preconditioning

Preconditioning (5 minutes of ischaemia followed by 10 minutes of recovery) renders the heart very resistant to infarction from subsequent ischaemia. The test method is disclosed in the article of G.S. Liu et al. in Circulation 84, 1, 350 to 356 (1991) . In this test 6-cyclohexyl-2' -O-methyl-adenosine hydrate is used in form of its 1.5 hydrate.

## Claims (Claims for the following Contracting State(s): AT, BE, CH, DE, DK, FR, GB, IT, LI, LU, NL, SE)

1. 6-cyclohexyl-2' -O-methyl-adenosine.hydrate.

2. 6-cyclohexyl-2' -O-methyl-adenosine hydrate according to claim 1 characterized in that the hydrate is a 1.5 hydrate.

3. Process for the preparation of 6-cyclohexyl-2' -O-methyl- adenosine hydrate according to claim 1 characterized in that 6-cyclohexyl-2' -O-methyl-adenosine-monoetherate is crystallized from an aqueous solvent.

4. Process for the production of the 1.5 hydrate of 6-cyclohexyl-2' -O-methyl-adenosine according to claim 2, characterized in that the 6-cyclohexyl-2' -O-methyl-adenosine-monoetherate is crystallized from a mixture of ethyl alcohol and water.

5. Use of 6-cyclohexyl-2' -O-methyl-adenosine hydrate for the preparation of medicaments suitable for the treatment of high blood pressure, as coronary vasodilators, for inhibition of both thrombocyte aggregation and activation of leucocytes, for lowering blood lipid levels, against congestive heart failure, myocardial infarction, sudden cardiac death, renal insufficiency further for the treatment of neurodegenerative disorders, certain peripheral neuropathies such as diabetic neuropathy and of disorders associated with peripheral vascular disease and/or disorders associated with neuronal degeneration, hypertriglyceridemia, elevated free fatty acids and/or type I and type II diabetes, arrhythmias and for protection against myocardial infarction.

6. A pharmaceutical composition comprising a therapeutically effective amount of 6-cyclohexyl-2' -O-methyl-adenosine hydrate and a pharmaceutically acceptable carrier.

7. A composition as claimed in claim 6 suitable for the treatment of high blood pressure, as coronary vasodilator, for inhibition of both thrombocyte aggregation and activation of leucocytes, for lowering blood lipid levels, against congestive heart failure, myocardial infarction, sudden cardiac death, renal insufficiency further for the treatment of neurodegenerative disorders, certain peripheral neuropathies such as diabetic neuropathy and of disorders associated with peripheral vascular disease and/or disorders associated with neuronal degeneration, hypertriglyceridemia, elevated free fatty acids and/or type I and type II diabetes, arrhythmias and for protection against myocardial infarction.

8. A composition as claimed in claim 6 or claim 7 wherein the hydrate is a 1.5 hydrate.

9. Use as claimed in claim 5 wherein the medicament is suitable for the treatment of neurodegenerative disorders, certain peripheral neuropathies such as diabetic neuropathy and of disorders associated with peripheral vascular disease and/or disorders associated with neuronal degeneration.

## Claims (Claims for the following Contracting State(s): ES, GR)

1. Process for the preparation of 6-cyclohexyl-2' -O-methyl-adenosine hydrate characterized in that 6-cyclohexyl-2' -O-methyl-adenosine-monoetherate is crystallized from an aqueous solvent.

2. Process for the production of the 1.5 hydrate of 6-cyclohexyl-2' -O-methyl-adenosine characterized in that the 6-cyclohexyl-2' -O-methyl-adenosine-monoetherate is crystallized from a mixture of ethyl alcohol and water.

3. Use of 6-cyclohexyl-2' -O-methyl-adenosine hydrate for the preparation of medicaments suitable for the treatment of high blood pressure, as coronary vasodilators, for inhibition of both thrombocyte aggregation and activation of leucocytes, for lowering blood lipid levels, against congestive heart failure, myocardial infarction, sudden cardiac death, renal insufficiency further for the treatment of neurodegenerative disorders, certain peripheral neuropathies such as diabetic neuropathy and of disorders associated with peripheral vascular disease and/or disorders associated with neuronal degeneration, hypertriglyceridemia, elevated free fatty acids and/or type I and type II diabetes, arrhythmias and for protection against myocardial infarction.

4. 6-cyclohexyl-2' -O-methyl-adenosine.hydrate.

5. 6-cyclohexyl-2' -O-methyl-adenosine hydrate according to claim 4 characterized in that the hydrate is a 1.5 hydrate.

6. A pharmaceutical composition comprising a therapeutically effective amount of 6-cyclohexyl-2' -O-methyl-adenosine hydrate and a pharmaceutically acceptable carrier.

7. A pharmaceutical composition as claimed in claim 6 comprising a therapeutically effective amount of 6-cyclohexyl-2' -O-methyl-adenosine hydrate and a pharmaceutically acceptable carrier thereof suitable for the treatment of high blood pressure, as coronary vasodilator, for inhibition of both thrombocyte aggregation and activation of leucocytes, for lowering blood lipid levels, against congestive heart failure, myocardial infarction, sudden cardiac death, renal insufficiency further for the treatment of neurodegenerative disorders, certain peripheral neuropathies such as diabetic neuropathy and of disorders associated with peripheral vascular disease and/or disorders associated with neuronal degeneration, hypertriglyceridemia, elevated free fatty acids and/or type I and type II diabetes, arrhythmias and for protection against myocardial infarction.

8. A composition as claimed in claim 6 or claim 7 wherein the hydrate is a 1.5 hydrate.

9. Use as claimed in claim 3 wherein the medicament is suitable for the treatment of neurodegenerative disorders, certain peripheral neuropathies such as diabetic neuropathy and of disorders associated with peripheral vascular disease and/or disorders associated with neuronal degeneration.

## Patentansprüche (Patentansprüche für folgende(n) Vertragsstaat(en): AT, BE, CH, DE, DK, FR, GB, IT, LI, LU, NL, SE)

1. 6-Cyclohexy-2'-O-methyladenosinhydrat.

2. 6-Cyxlohexy-2'-O-methyladenosinhydrat nach Anspruch 1, dadurch gekennzeichnet, daß das Hydrat ein 1,5 Hydrat ist.

3. Verfahren zur Herstellung von 6-Cyclohexyl-2'-O-methyladenosinhydrat nach Anspruch 1, dadurch gekennzeichnet, daß 6-Cyclohexyl-2'-O-methyladenosinmonoetherat aus einem wäßrigen Lösemittel kristallisiert wird.

4. Verfahren zur Herstellung des 1,5 Hydrats von 6-Cyclohexyl-2'-O-methyladenosin nach Anspruch 2, dadurch gekennzeichnet, daß 6-Cyclohexyl-2'-O-methyladenosinmonoetherat aus einem Gemisch aus Ethylalkohol und Wasser kristallisiert wird.

5. Verwendung von 6-Cyclohexyl-2'-O-rnethyladenosinhydrat zur Herstellung von Arzneimitteln, die geeignet sind zur Behandlung von hohem Blutdruck, wie Koronarvasodilatatoren, zur Hemmung sowohl der Thrombozytenaggregation als auch der Aktivierung von Leukozyten, zur Absenkung der Blutlipidspiegel, gegen angeborene Herzschwäche, Myokardinfarkt, plötzlichen Herztod, Niereninsuffizienz, ferner zur Behandlung von neurodegenerativen Störungen, bestimmten peripheren Neuropathien, wie dibetische Neuropathie und Störungen, die mit einer peripheren vaskulären Erkrankung zusammenhängen und/oder Störungen, die mit einer neuronalen Degeneration zusammenhängen, Hypertriglyceridämie, erhöhte freie Fettsäuren und/oder Typ I und Typ II Diabetes, Arrhythmien und zum Schutz gegen Myokardinfarkt.

6. Pharmazeutische Zusammensetzung, die eine therapeutisch wirksame Menge 6-Cyclohexyl-2'-O-methyladenosinhydrat und einen pharmazeutisch annehmbaren Träger enthält.

7. Zusammensetzung nach Anspruch 6, die geeignet ist zur Behandlung von hohem Blutdruck, wie Koronarvasodilatatoren, zur Hemmung sowohl der Thrombozytenaggregation als auch der Aktivierung von Leukozyten, zur Absenkung der Blutlipidspiegel, gegen angeborene Herzschwäche, Myokardinfarkt, plötzlichen Herztod, Niereninsuffizienz, ferner zur Behandlung von neurodegenerativen Störungen, bestimmten peripheren Neuropathien, wie diabetische Neuropathie und Störungen, die mit einer peripheren vaskulären Erkrankung zusammenhängen und/oder Störungen, die mit einer neuronalen Degeneration zusammenhängen, Hypertriglyceridämie, erhöhte freie Fettsäuren und/oder Typ I und Typ II Diabetes, Arrhythmien und zum Schutz gegen Myokardinfarkt.

8. Zusammensetzung nach Anspruch 6 oder 7, worin das Hydrat ein 1,5 Hydrat ist.

9. Verwendung nach Anspruch 5, worin das Arzneimittel geeignet ist zur Behandlung von neurodegenerativen Störungen, bestimmten peripheren Neuropathien, wie diabetische Neuropathie und Störungen, die mit einer peripheren vaskulären Erkrankung zusammenhängen und/oder Störungen, die mit einer neuronalen Degeneration zusammenhängen.

## Patentansprüche (Patentansprüche für folgende(n) Vertragsstaat(en): ES, GR)

1. Verfahren zur Herstellung von 6-Cyclohexyl-2'-O-methyladenosinhydrat, dadurch gekennzeichnet, daß 6-Cyclohexyl-2'-O-methyladenosinmonoetherat aus einem wäßrigen Lösemittel kristallisiert wird.

2. Verfahren zur Herstellung des 1,5 Hydrats von 6-Cyclohexyl-2'-O-methyladenosin, dadurch gekennzeichnet, daß 6-Cyclohexyl-2'-O-methyladenosinmonoetherat aus einem Gemisch aus Ethylalkohol und Wasser kristallisiert wird.

3. Verwendung von 6-Cyclohexyl-2'-O-methyladenosinhydrat zur Herstellung von Arzneimitteln, die geeignet sind zur Behandlung von hohem Blutdruck, wie Koronarvasodilatatoren, zur Hemmung sowohl der Thrombozytenaggregation als auch der Aktivierung von Leukozyten, zur Absenkung der Blutlipidspiegel, gegen angeborene Herzschwäche, Myokardinfarkt, plötzlichen Herztod, Niereninsuffizienz, ferner zur Behandlung von neurodegenerativen Störungen, bestimmten peripheren Neuropathien, wie diabetische Neuropathie und Störungen, die mit einer peripheren vaskulären Erkrankung zusammenhängen und/oder Störungen, die mit einer neuronalen Degeneration zusammenhängen, Hypertriglyceridämie, erhöhte freie Fettsäuren und/oder Typ I und Typ II Diabetes, Arrhythmien und zum Schutz gegen Myokardinfarkt.

4. 6-Cyclohexyl-2'-O-methyladenosinhydrat.

5. 6-Cyclohexyl-2'-O-methyladenosinhydrat nach Anspruch 4, dadurch gekennzeichnet, daß das Hydrat ein 1,5 Hydrat ist.

6. Pharmazeutische Zusammensetzung, die eine therapeutisch wirksame Menge 6-Cyclohexyl-2'-O-methyladenosinhydrat und einen pharmazeutisch annehmbaren Träger enthält.

7. Pharmazeutische Zusammensetzung nach Anspruch 6, die eine therapeutisch wirksame Menge von 6-Cyclohexyl-2'-O-methyladenosinhydrat und einen pharmazeutisch annehmbaren Träger enthält und geeignet ist zur Behandlung von hohem Blutdruck, wie Koronarvasodilatatoren, zur Hemmung sowohl der Thrombozytenaggregation als auch der Aktivierung von Leukozyten, zur Absenkung der Blutlipidspiegel, gegen angeborene Herzschwäche, Myokardinfarkt, plötzlichen Herztod, Niereninsuffizienz, ferner zur Behandlung von neurodegenerativen Störungen, bestimmten peripheren Neuropathien, wie diabetische Neuropathie und Störungen, die mit einer peripheren vaskulären Erkrankung zusammenhängen und/oder Störungen, die mit einer neuronalen Degeneration zusammenhängen, Hypertriglyceridämie, erhöhte freie Fettsäuren und/oder Typ I und Typ II Diabetes, Arrhythmien und zum Schutz gegen Myokardinfarkt.

8. Zusammensetzung nach Anspruch 6 oder 7, worin das Hydrat ein 1,5 Hydrat ist.

9. Verwendung nach Anspruch 3, worin das Arzneimittel geeignet ist zur Behandlung von neurodegenerativen Störungen, bestimmten peripheren Neuropathien, wie diabetische Neuropathie und Störungen, die mit einer peripheren vaskulären Erkrankung zusammenhängen und/oder Störungen, die mit einer neuronalen Degeneration zusammenhängen.

## Revendications (Revendications pour l'(les) Etat(s) contractant(s) suivant(s): AT, BE, CH, DE, DK, FR, GB, IT, LI, LU, NL, SE)

1. Hydrate de la 6-cyclohexyl-2'-O-méthyl-adénosine.

2. Hydrate de la 6-cyclohexyl-2'-O-méthyl-adénosine selon la revendication 1, caractérisé en ce que l'hydrate est un 1,5 hydrate.

3. Un procédé de préparation de l'hydrate de la 6-cyclohexyl-2'-O-méthyl-adénosine selon la revendication 1, caractérisé en ce qu'on cristallise le monoéthérate de la 6-cyclohexyl-2'-O-méthyl-adénosine dans un solvant aqueux.

4. Un procédé de préparation du 1,5 hydrate de la 6-cyclohexyl-2'-O-méthyl-adénosine selon la revendication 2, caractérisé en ce qu'on cristallise le monoéthérate de la 6-cyclohexyl-2'-O-méthyl-adénosine dans un mélange d'alcool éthylique et d'eau.

5. L'utilisation de l'hydrate de la 6-cyclohexyl-2'-O-méthyl-adénosine pour la préparation de médicaments appropriés pour le traitement de la tension artérielle élevée, comme vasodilatateur coronarien, pour l'inhibition de l' agrégation plaquettaire et de l'activation des leucocytes, pour l'abaissement des taux de lipides dans le sang, contre l'insuffisance cardiaque globale, l'infarctus du myocarde, la mort cardiaque subite, l'insuffisance rénale et également pour le traitement de troubles neurodégénératifs, de certaines neuropathies du système nerveux périphérique telles que la neuropathie diabétique et des troubles associés à une maladie vasculaire périphérique et/ou des troubles associés à une dégénérescence neuronale, de l'hypertriglycéridémie, des taux élevés d'acides gras libres et/ou du diabète du type I et du type II, des arhytmies et pour la protection contre l'infarctus du myocarde.

6. Une composition pharmaceutique comprenant une quantité efficace du point de vue thérapeutique de l'hydrate de la 6-cyclohexyl-2'-O-méthyl-adénosine et un véhicule pharmaceutiquement acceptable.

7. Une composition selon la revendication 6 appropriée pour le traitement de la tension artérielle élevée, comme vasodilatateur coronarien, pour l'inhibition de l'agrégation plaquettaire et de l'activation des leucocytes, pour l'abaissement des taux de lipides dans le sang, contre l'insuffisance cardiaque globale, l'infarctus du myocarde, la mort cardiaque subite, l'insuffisance rénale et également pour le traitement de troubles neurodégénératifs, de certaines neuropathies du système nerveux périphérique telles que la neuropathie diabétique et des troubles associés à une maladie vasculaire périphérique et/ou des troubles associés à une dégénérescence neuronale, de l'hypertriglycéridémie, des taux élevés d'acides gras libres et/ou du diabète du type I et du type II, des arhytmies et pour la protection contre l'infarctus du myocarde.

8. Une composition selon la revendication 6 ou 7, où l'hydrate est un 1,5-hydrate.

9. L'utilisation selon la revendication 5, où le médicament est approprié pour le traitement de troubles neurodégénératifs, de certaines neuropathies du système nerveux périphérique telles que la neuropathie diabétique et des troubles associés à une maladie vasculaire périphérique et/ou des troubles associés à une dégénérescence neuronale.

## Revendications (Revendications pour l'(les) Etat(s) contractant(s) suivant(s): ES, GR)

1. Un procédé de préparation de l'hydrate de la 6-cyclohexyl-2'-O-méthyl-adénosine, caractérisé en ce qu'on cristallise le monoéthérate de la 6-cyclohexyl-2'-O-méthyl-adénosine dis un solvant aqueux.

2. Un procédé de préparation du 1,5 hydrate de la 6-cyclohexyl-2'-O-méthyl-adénosine, caractérisé en ce qu'on cristallise le monoéthérate de la 6-cyclohexyl-2'-O-méthyl-adénosine dans un mélange d'alcool éthylique et d'eau.

3. L'utilisation de l'hydrate de la 6-cyclohexyl-2'-O-méthyl-adénosine pour la préparation de médicaments appropriés pour le traitement de la tension artérielle élevée, comme vasodilatateur coronarien, pour l'inhibition de l'agrégation plaquettaire et de l'activation des leucocytes, pour l'abaissement des taux de lipides dans le sang, contre l'insuffisance cardiaque globale, l'infarctus du myocarde, la mort cardiaque subite, l'insuffisance rénale et également pour le traitement de troubles neurodégénératifs, de certaines neuropathies du système nerveux périphérique telles que la neuropathie diabétique et des troubles associés à une maladie vasculaire périphérique et/ou des troubles associés à une dégénérescence neuronale, de l'hypertriglycéridémie, des taux élevés d'acides gras libres et/ou du diabète du type I et du type II, des arhytmies et pour la protection contre l'infarctus du myocarde.

4. Hydrate de la 6-cyclohexyl-2'-O-méthyl-adénosine.

5. Hydrate de la 6-cyclohexyl-2'-O-méthyl-adénosine selon la revendication 4, caractérisé en ce que l'hydrate est un 1,5 hydrate.

6. Une composition pharmaceutique comprnant une quantité efficace du point de vue thérapeutique de l'hydrate de la 6-cyclohexyl-2'-O-méthyl-adénosine et un véhicule pharmaceutiquement acceptable.

7. Une composition pharmaceutique selon la revendication 6, comprenant une quantité efficace du point de vue thérapeutique de l'hydrate de la 6-cyclohexyl-2'-O-méthyl-adénosine et un véhicule pharmaceutiquement acceptable de ce dernier appropriée pour le traitement de la tension artérielle élevée, comme vasodilatateur coronarien, pour l'inhibition de l'agrégation plaquettaire et de l'activation des leucocytes, pour l'abaissement des taux de lipides dans le sang, contre l'insuffisance cardiaque globale, l'infarctus du myocarde, la mort cardiaque subite, l'insuffisance rénale et également pour le traitement de troubles neurodégénératifs, de certaines neuropathies du système nerveux périphérique telles que la neuropathie diabétique et des troubles associés à une maladie vasculaire périphérique et/ou des troubles associés à une dégénérescence neuronale, de l'hypertriglycéridémie, des taux élevés d'acides gras libres et/ou du diabète du type I et du type II, des arhytmies et pour la protection contre l'infarctus du myocarde.

8. Une composition selon la revendication 6 ou 7, où l'hydrate est un 1,5 hydrate.

9. L'utilisation selon la revendication 3, où le médicament est approprié pour le traitement de troubles neurodégénératifs, de certaines neuropathies du système nerveux périphérique telles que la neuropathie diabétique et des troubles associés à une maladie vasculaire périphérique et/ou des troubles associés à une dégénérescence neuronale.
